# EUROPÄISCHE PATENTSCHRIFT

(11) **EP 1 171 445 B1**
(45) Veröffentlichungstag und Bekanntmachung des Hinweises auf die Patenterteilung: **21.01.2004**
(21) Anmeldenummer: 00920583.2
(22) Anmeldetag: 25.03.2000
(51) Int. Cl.: C07F 11/00, C07C 29/16, C07C 2/32, B01J 31/18

(54) **OLIGOMERISIERUNGSKATALYSATOR**
OLIGOMERISATION CATALYST
CATALYSEUR D'OLIGOMERISATION

(30) Priorität: 29.03.1999 US 277823; 14.05.1999 DE 19922048; 11.09.1999 DE 19943544
(43) Veröffentlichungstag der Anmeldung: 16.01.2002
(73) Patentinhaber: BASF AKTIENGESELLSCHAFT, 67056 Ludwigshafen (DE)
(72) Erfinder: MAAS, Heiko, D-67105 Schifferstadt (DE); MIHAN, Shahram, D-67061 Ludwigshafen (DE); KÖHN, Randolf, Bath BA2 2AZ (GB); SEIFERT, Guido, D-10623 Berlin (DE); TROPSCH, Jürgen, D-67354 Römerberg (DE)
(74) Vertreter: Pohl, Michael, Dr.
(86) Internationale Anmeldenummer: PCT/EP2000/002660
(87) Internationale Veröffentlichungsnummer: WO 2000/058319

(56) Entgegenhaltungen:
- EP-A- 0 537 609
- WO-A-00/34211
- CHEMICAL ABSTRACTS, vol. 129, no. 18, 2. November 1998 (1998-11-02) Columbus, Ohio, US; abstract no. 231170, TANI, KAZUHIDE ET AL: "Preparation of.alpha.-olefin polymers by the use of vanadium or chromium complex catalysts" XP002139748 & JP 10 231317 A (MITSUBISHI CHEMICAL INDUSTRIES LTD., JAPAN) 2. September 1998 (1998-09-02)

## Beschreibung

Die vorliegende Erfindung betrifft einen Oligomerisierungskatalysator für Olefine, erhältlich aus
a) einer Chromverbindung CrX₃ und der mindestens äquimolaren Menge, bezogen auf die Chromverbindung CrX₃, eines Liganden L oder einem fertigen Chromkomplex CrX₃L, in denen die Gruppen X unabhängig voneinander für abstrahierbare Gegenionen stehen und L für ein 1,3,5-Triazacyclohexan der Formel I steht 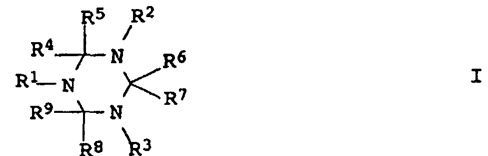 in welcher die Gruppen R¹ bis R⁹ unabhängig voneinander folgende Bedeutungen haben: Wasserstoff oder Si-organische oder gegebenenfalls substituierte C-organische Gruppen mit 1 bis 30 C-Atomen, wobei zwei geminale oder vicinale Reste R¹ bis R⁹ auch zu einem fünf- oder sechsgliedrigen Ring verbunden sein können und
b) mindestens einem aktivierenden Zusatzstoff.

Des Weiteren betrifft die vorliegende Erfindung ein Verfahren zur Herstellung von Oligomeren von Olefinen unter Verwendung derartiger Katalysatoren.

Olefinoligomere mit bis zu 30 Kohlenstoffatomen haben große wirtschaftliche Bedeutung als Copolymere für Kunststoffe (z.B. 1-Hexen) bzw. als Vorprodukte für Oxoalkohole (z.B. 1-Hexen sowie die Decene und Tetradecene), wobei letztere wiederum Bestandteil von Tensiden und Weichmachern für Kunststoffe sind. Im Produktverbund der chemischen Industrie sind damit die Oligomerisierungsverfahren ein zentraler Schritt von den großtechnischen Olefinströmen, die etwa den Steamcrackern entstammen, zu Produkten des täglichen Bedarfs.

Die Verwendung von Katalysatoren, welche Verbindungen des Chrom, Amine und Aluminiumverbindungen enthalten, bei der Oligomerisierung von α-Olefinen ist allgemein bekannt:

Die JP 10231317 beschreibt ein Verfahren zur Herstellung von Polymeren aus α-Olefinen in Gegenwart eines Katalysators, der neben einem Aluminiumalkyl einen Vanadium- oder Chromkomplex mit einem substituierten oder unsubstituierten 1,3,5-Triazacyclohexan-Liganden umfasst.

Die WO 00/34211 offenbart Komplexe von Chromverbindungen mit 1,3,5-Triazacyclohexan-Liganden und deren Verwendung für die Oligomerisierung von C₈-C₃₆-α-Olefinen. Zur Aktivierung der Katalysatoren werden Alkylalumoxane vorgeschlagen.

Gemäß der EP-A 780 353 lassen sich Olefine in Gegenwart einer Chromquelle, einer Pyrrol-enthaltenden Verbindung und einem Metallalkyl oligomerisieren, insbesondere trimerisieren. Die Vorbereitung des Katalysators geht jedoch mit einem Verlust an aktiven Bestandteilen einher.

Aus der DE-A 196 07 888 ist ein Oligomerisierungskatalysator für α-Olefine bekannt, der neben einer Chromverbindung und einer Aluminiumverbindung noch mindestens eine Stickstoff enthaltende Verbindung, die ein Pyrrol sein kann, enthält. Auch hier ist die Katalysatorvorbereitung entsprechend der EP-A 780 353 verlustreich.

Die EP-A 537 609 lehrt ein Verfahren, bei dem Ethylen in Gegenwart eines Chromkomplexes mit einem koordinierenden Polydentatliganden und einem Aluminoxan zu einem Gemisch von α-Olefinen mit einem erhöhten Anteil an 1-Hexen umgesetzt wird. Wegen der niedrigen Katalysatoraktivität bei gleichzeitig niedriger Trimerenselektivität ist jedoch die Wirtschaftlichkeit des beschriebenen Verfahrens unbefriedigend.

Auf dem 215. ACS National Meeting, 29. März - 2. April 1998 in Dallas, Texas, wurde über Versuche zur selektiven Trimerisierung von Ethylen zu 1-Hexen mit einem N,N,N-Trioctyl-triazacyclohexan-Chromkomplex und Methylalumoxan berichtet. Jedoch ist der Katalysator nur mäßig aktiv und er führt noch zu erheblichen Mengen an polymeren Produkten,was für die Wirtschaftlichkeit des Verfahrens von Nachteil ist.

Der vorliegenden Erfindung lagen daher kostengünstiger erhältliche und beständige Katalysatoren mit verbesserter Aktivität und Selektivität bezüglich niedermolekularen Oligomeren von Olefinen als Aufgabe zugrunde.

Demgemäß wurden Oligomerisierungskatalysatoren der Eingangs definierten Art gefunden, die neben der Chromverbindung CrX₃ als Komponente a) wenigstens einen aktivierenden Zusatzstoff aus der Gruppe:
i) ein gegebenenfalls substituierter fünfgliedriger aromatischer N-Heterocyclus und mindestens ein Aluminiumalkyl, dessen Alkylgruppen teilweise durch Halogen und/oder Alkoxy ersetzt sein können,
ii) ein Alkylalumoxan
umfassen.

Gegenstand der Erfindung ist somit ein Verfahren zur Herstellung von Oligomeren mit bis zu 30 Kohlenstoffatomen durch Umsetzung eines C₂-C₄-Olefins oder eines Gemischs von C₂-C₄-Olefinen bei Temperaturen von 0 bis 150°C und Drücken von 1 bis 200 bar in Gegenwart eines Oligomerisierungskatalysators, der erhältlich ist aus
a) einer Chromverbindung CrX₃ und der mindestens äquimolaren Menge, bezogen auf die Chromverbindung CrX₃, eines Liganden L oder einem fertigen Chromkomplex CrX₃L, in denen die Gruppen X unabhängig voneinander für abstrahierbare Gegenionen stehen und L für ein 1,3,5-Triazacyclohexan der Formel I steht 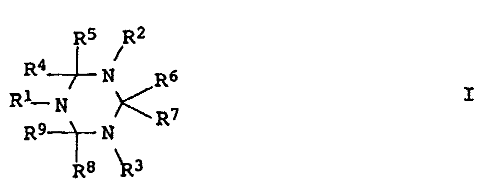 in welcher die Gruppen R¹ bis R⁹ unabhängig voneinander folgende Bedeutungen haben: Wasserstoff oder Si-organische oder gegebenenfalls substituierte C-organische Gruppen mit 1 bis 30 C-Atomen, wobei zwei geminale oder vicinale Reste R¹ bis R⁹ auch zu einem fünf- oder sechsgliedrigen Ring verbunden sein können und
b) mindestens einem aktivierenden Zusatzstoff aus der Gruppe:
   i) ein gegebenenfalls substituierter fünfgliedriger aromatischer N-Heterocyclus und mindestens ein Aluminiumalkyl, dessen Alkylgruppen teilweise durch Halogen und/oder Alkoxy ersetzt sein können,
   ii) ein Alkylalumoxan.

Gegenstand der Erfindung sind auch derartige Katalysatoren, die als aktivierenden Zusatzstoff b) einen gegebenenfalls substituierten fünfgliedrigen aromatischen N-Heterocyclus und mindestens ein Aluminiumalkyl, dessen Alkylgruppen teilweise durch Halogen und/oder Alkoxy ersetzt sein können, enthalten.

Mit den erfindungsgemäßen Oligomerisierungskatalysatoren lassen sich Oligomere von Olefinen mit hohen Ausbeuten und mit einem geringen Anteil an Nebenprodukten, deren Molmasse M_{W} größer ist als 500, gewinnen. Insbesondere zeichnet sich der Katalysator durch eine hohe Selektivität hinsichtlich der Trimerisierung von α-Olefinen und vor allem von Ethen, aus.

Triazacyclohexan und seine Derivate, die sich durch unterschiedliche Substitutionsmuster an den Ringatomen unterscheiden, sind seit langem bekannt und werden technisch vielseitig verwendet, da sie zumeist aus gut verfügbaren Ausgangsprodukten in einfacher und kostengünstiger Weise herstellbar sind. So werden Triazacyclohexan-Derivate beispielsweise bei der Entschwefelung von Kerosin eingesetzt. Die Verwendung von Triazacyclohexan und seiner Derivate als Liganden bei der Herstellung metallorganischer Komplexe ist jedoch kaum verbreitet. Nur vereinzelt werden in der metallorganischen Literatur Komplexe mit diesen Liganden beschrieben, so beispielsweise in J. Chem. Soc., Dalton Trans. (1997), 1363-1368; Z. Naturforsch., Teil B50 (1995), 1038-1043; Angew. Chem. Int. Ed. Engl. 33 (1994), 1877-1878; J. Organomet. Chem. 501 (1995), 303-307; Chem. Ber. 129 (1996), 25-27; J. Organomet. Chem. 520 (1996), 121-129; Inorg. Chem. 36 (1997), 6064-6069; Chem. Ber. 129 (1996), 1327-1333.

Durch die Variation der Substituenten am 1,3,5-Triazacyclohexanring lassen sich die Eigenschaften des erfindungsgemäßen Katalysators beeinflussen. So läßt sich die Katalysatoraktivität durch Substituenten, insbesondere an den Stickstoffatomen, normalerweise steigern. Durch die Anzahl und Geometrie der Substituenten läßt sich weiterhin die Zugänglichkeit des Zentralatoms für die umzusetzenden α-Olefine steuern und damit auch die Selektivität der Umsetzung bezüglich verschiedener Ausgangs-Olefine. Die chemische Struktur der Substituenten R¹ bis R⁹ kann daher in weiten Bereichen variiert werden, um einen für die jeweilige Umsetzung maßgeschneiderten Katalysator zu erhalten.

Als gegebenenfalls substituierte C-organische Gruppen am 1,3,5-Triazacyclohexanring kommen beispielsweise in Betracht:
- C₁- bis C₁₈-Alkyl, vorzugsweise C₁- bis C₁₂-Alkyl wie Methyl, Ethyl, N,N-Dimethylaminoethyl, n-Propyl, i-Propyl, Butyl, Pentyl, Hexyl, 2-Ethylhexyl, Heptyl, Octyl, Nonyl, Decyl, Dodecyl, 1,1-Dimethyldodecyl,
- 5- bis 7-gliedriges Cycloalkyl, das seinerseits eine C₁⁻ bis C₁₀-Arylgruppe als Substituent tragen kann wie Cyclopentyl und Cyclohexyl,
- C₆- bis C₁₅-Aryl wie Phenyl, o-Tolyl, p-Tolyl, m-Tolyl, 1-Naphthyl und 2-Naphthyl oder
- C₇- bis C₁₅-Arylalkyl, vorzugsweise C₇- bis C₈-Arylalkyl wie Benzyl und (1-Phenyl)ethyl.

Als Si-organische Gruppen kommen beispielsweise in Betracht: Trialkylsilyl-Gruppen mit 1 bis 10 C-Atomen in gleichen oder verschiedenen Alkylresten, insbesondere Trimethylsilyl-Gruppen.

Substituenten in den C-organische Gruppen oder Si-organische Gruppen am 1,3,5-Triazacyclohexanring können insbesondere Alkylgruppen mit Donorgruppen sein. Die Donorgruppe kann neutral oder anionisch sein und ein Heteroatom der Gruppen 15-16 des Periodensystems (nach IUPAC Vorschlag 1985) enthalten oder ein Carbanion sein. Ist sie neutral so kann sie koordinativ an das Chrom gebunden sein. Bevorzugt ist sie an das Chrom koordiniert. Ist der Donor formal anionisch so ist er kovalent an das Metallzentrum gebunden. Die Bindungen können intra- oder intermolekular sein, bevorzugt sind sie intramolekular. Bevorzugt sind neutrale Donoren mit Sauerstoff- und/oder Stickstoffatomen, die freie Elektronenpaare aufweisen, wobei die Sauerstoff- und/oder Stickstoffatome auch in eine Alkylkette eingefügt sein können.

Bevorzugt sind 1,3,5-Triazacyclohexanliganden, in denen die Gruppen R¹, R² und R³ unabhängig voneinander für gegebenenfalls substituiertes C₁- bis C₁₂-Alkyl, C₆- bis C₁₅-Aryl oder C₇- bis C₈-Arylalkyl stehen, insbesondere gegebenenfalls substituiertes C₁- bis C₁₂-Alkyl oder C₇- bis C₈-Arylalkyl wie Methyl, Ethyl, N,N-Dimethylaminoethyl, n-Propyl, n-Butyl, tert.-Butyl, Hexyl, Octyl, Dodecyl, 1,1-Dimethyldodecyl, (1-Phenyl)ethyl.

Im erfindungsgemäßen Verfahren verwendet man besonders bevorzugt 1,3,5-Triazacyclohexanliganden, in denen die Gruppen R⁴, R⁵, R⁶, R⁷, R⁸ und R⁹ unabhängig voneinander für Wasserstoff oder C₁- bis C₄-Alkyl und insbesondere Wasserstoff oder Methyl stehen.

Bevorzugte 1,3,5-Triazacyclohexane sind 1,3,5-Tri-tert.-butyl-1,3,5-triazacyclohexan, 1,3,5-Triethyl-1,3,5-triazacyclohexan, 1,3,5-Tris-[(1-phenyl)ethyl]-1,3,5-triazacyclohexan, 1,3,5-Tris-[(1,1-dimethyl)dodecyl]-1,3,5-triazacyclohexan und 1,3-Di-n-dodecyl-5-[2-(N,N-Dimethylamino)ethyl]-1,3,5-triazacyclohexan sowie besonders bevorzugt 1,3,5-Tri-n-octyl-1,3,5-triazacyclohexan, 1,3,5-Tri-n-dodecyl-1,3,5-triazacyclohexan, 1,3,5-Tribenzyl-1,3,5-triazacyalohexan, 1,3,5-Tris-(2-ethylhexyl)-1,3,5-triazacyclohexan, 1,3,5-Tris-(2-n-propylheptyl)-1,3,5-triazacyclohexan.

Die 1,3,5-Triazacyclohexane der allgemeinen Formel I, in denen die Gruppen R⁴ bis R⁹ für Wasserstoff stehen und die Gruppen R¹ bis R³ gleich sind, lassen sich in an sich bekannter Weise herstellen, beispielsweise durch Umsetzung primärer Amine vom Typ R¹NH₂ mit Formaldehyd oder Paraf ormaldehyd. Entsprechend sind die 1,3,5-Triazacyclohexane, welche jeweils eine Methylgruppe und ein Wasserstoffatom an den Kohlenstoffatomen des Rings tragen, über Acetaldehyd zugänglich.

Auch die 1,3,5-Triazacyclohexane der allgemeinen Formel I, in denen mindestens einer der Reste R¹, R² oder R³ von den übrigen dieser Reste unterschiedlich ist, können in an sich bekannter Weise hergestellt werden (vgl. etwa Beilstein, "Handbook of Organic Chemistry", 4th Ed., Vth Suppl. Series, Springer-Verlag, Berlin, Vol. 26 (1986) 3 ff. u. die dort zitierten Referate; R = Octyl: J. Polym. Sci., Polym. Chem. Ed. 329 (1993), 1941-1958; J. Prakt. Chem. 327 (1985), 739-748; EP-A 620 266; DE-A 24 31 862; DE-A 41 00 856; Pharmazie 30 (1975), 699-706). Beispielhaft seien hier einige der bekannten Herstellverfahren kurz skizziert:
1) Die Umsetzung eines Gemisches aus zwei primären Aminen (R¹NH₂ und R²NH₂) mit Formaldehyd (als wäßrige Lösung oder Paraformaldehyd) führt einem Gemisch verschiedener Produkte, die wie folgt getrennt werden können:
   a) Destillation bei ausreichend kleinen R¹ und R².
   b) Durchführung der Reaktion mit einem großen Überschuß an dem Amin R¹NH₂, wenn das symmetrische Reaktionsprodukt abdestillierbar ist. Nach Destillation verbleibt dann das unsymmetrische Produkt.
   c) Selektive Kristallisation eines Produktes.
   d) Komplexierung des Gemisches der 1,3,5-Triazacyclohexane mit der Chromverbindung, etwa CrCl₃, und Trennung der so erhaltenen Chrom-Komplexe etwa durch Säulenchromatographie.
2) Umsetzung eines Amins R¹NH₂ mit einem Überschuß an Formaldehyd zu einem Gemisch aus symmetrisch substituiertem 1,3,5-Triazacyclohexan und dem entsprechenden 1-Oxa-3,5-diazacyclohexan:
   In einem zweiten Schritt wird das 1-Oxa-3,5-diazacyclohexan mit einem Amin R²NH₂ (ggf. unter Säurekatalyse) umgesetzt, wobei der Ringsauerstoff gegen eine Gruppe R²N ausgetauscht wird. 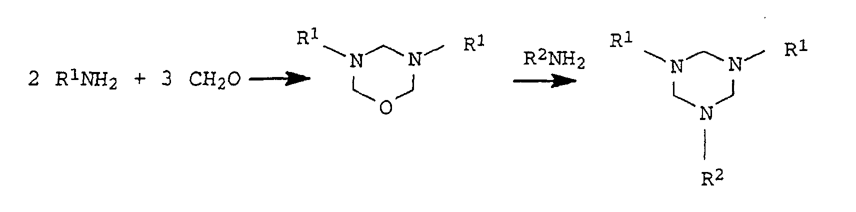
   Die Trennung des Produktgemischs kann wie unter 1) erfolgen.
3) Umsetzung eines symmetrischen 1,3,5-Triazacyclohexans mit kleinem R¹ (Methyl oder Ethyl) bei ca. 130°C mit einem Amin R²NH₂. Bei dieser Temperatur entweicht R¹NH₂ und ein Gemisch der unsymmetrischen 1,3,5-Triazacyclohexane wird gebildet. 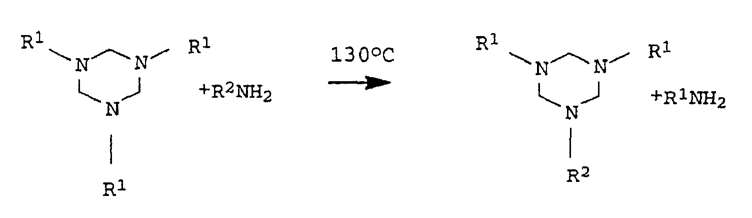 Die Trennung kann wie unter 1) erfolgen.
4) Umsetzung zweier verschiedener symmetrischer 1,3,5-Triazacyclohexane miteinander, wobei Substituentenaustausch eintritt. Die Produkte können wie unter 1) getrennt werden.

Als Gruppen X in den Chromverbindungen CrX₃ oder den Chromkomplexen CrX₃L eignen sich jegliche in der metallorganischen Chemie zu diesem Zweck geeignete abstrahierbare Gegenionen, insbesondere
- Halogen wie Fluor, Brom, Jod und vor allem Chlor,
- Tosylat, Triflat, Tetrafluoroborat, Hexafluorophosphat, Hexafluoroantimonat, Tetraphenylborat,
- C₁- bis C₁₀-Carboxy, vor allem 2-Ethylhexanoat,
- Alkylgruppen, beispielsweise Methyl, Ethyl, i-Propyl, Phenyl Benzyl,
- sperrige nichtkoordinierende Anionen wie B(C₆F₅)₄⁻.

Die Gruppen X wählt man insbesondere so aus, dass die sie enthaltenden Chromverbindungen CrX₃ bzw. Chromkomplexe CrX₃L eine gute Löslichkeit im jeweils verwendeten Lösungsmittel haben.

Man verwendet als Ausgangsstoffe für die Chromverbindungen CrX₃ und die Chromkomplexe CrX₃L etwa Chromhalogenide wie CrCl₃, CrBr₃, Cr(Triflat)₃, Cr-(III)-Alkoxylate wie das 2-Ethylhexanoat sowie Komplexe dieser Chromverbindungen mit schwach gebundenen neutralen Komplexliganden, die durch das 1,3,5-Triazacyclohexan und gegebenenfalls durch den fünfgliedrige aromatische N-Hetereocyclus verdrängt werden können, z.B. Etherkomplexe wie CrCl₃(Tetrahydrofuran)₃, CrCl₃(Dioxan)₃, Esterkomplexe wie CrCl₃(n-Butylacetat), CrCl₃(Ethylacetat), Alkoholkomplexe wie CrCl₃(i-Propanol)₃, CrCl₃(2-Ethylhexanol)₃, Aminkomplexe wie CrCl₃(Pyridin)₃, CrCl₃(i-Propylamin)₂, oder Nitrilkomplexe wie CrCl₃(Acetonitril)₃·Acetonitril.

In einer bevorzugten Ausführungsform des erfindungsgemäßen Verfahrens kommen Oligomerisierungskatalysatoren zum Einsatz, welche man unter Verwendung von zuvor eigens hergestellten und isolierten Chromkomplexen CrX₃L hergestellt hat.

Die Chromkomplexe CrX₃L sind im übrigen nach dem Fachmann bekannten Methoden oder analog zu diesen erhältlich (vgl. etwa W. A. Herrmann, A. Salzer: "Synthetic Methods of Organometallic and Inorganic Chemistry", Vol. 1 - Literature, Laboratory Techniques, and Common Starting Materials, Thieme Verlag, Stuttgart, 1996).

Bei der Herstellung der Chromkomplexe CrX₃L in situ geht man in der Regel so vor, dass man die Chromverbindung CrX₃ im Reaktionsmedium löst oder suspendiert und das 1,3,5-Triazacyclohexan wahlweise in Substanz oder in gelöster Form zugibt.

Im erfindungsgemäßen Oligomerisierungsverfahren ist die Verwendung von Chromkomplexen CrX₃L bevorzugt, in denen X und L die folgenden Bedeutungen haben:
- X: unabhängig voneinander: Halogen, Tosylat, Triflat, Alkyl
- L: 1,3,5-Triazacyclohexane der Formel I, in denen die Gruppen R⁴, R⁵, R⁶, R⁷, R⁸ und R⁹ unabhängig voneinander für Wasserstoff oder C₁- bis C₄-Alkyl und insbesondere Wasserstoff oder Methyl stehen und in denen R¹, R² und R³ unabhängig voneinander für Methyl, Ethyl, N,N-Dimethylaminoethyl, Propyl, n-Butyl, tert.-Butyl, Hexyl, Octyl, Dodecyl, 1,1-Dimethyldodecyl oder (1-Phenyl)ethyl stehen.

Besonders bevorzugt ist die Verwendung von Chromkomplexen CrX₃L, in denen X und L die folgenden Bedeutungen haben:
- X: unabhängig voneinander: Chlor, Tosylat
- L: 1,3,5-Triazacyclohexane der Formel I, in denen die Gruppen R⁴, R⁵, R⁶, R⁷, R⁸ und R⁹ unabhängig voneinander für Wasserstoff oder Methyl stehen und in denen R¹, R² und R³ unabhängig voneinander für Methyl, Ethyl, N,N-Dimethylaminoethyl, Propyl, n-Butyl, tert.-Butyl, Hexyl, Octyl, Dodecyl, 1,1-Dimethyldodecyl oder (2-Phenyl)ethyl stehen.

In einer bevorzugten Ausführungsform des erfindungsgemäßen Verfahrens setzt sich der aktivierende Zusatzstoff zusammen aus einem gegebenenfalls substituierten fünfgliedrigen aromatischen N-Heterocyclus und mindestens einem Aluminiumalkyl, dessen Alkylgruppen teilweise durch Halogen und/oder Alkoxy ersetzt sein können.

Geeignete fünfgliedrige aromatische N-Hetereocyclen sind solche mit 1, 2, 3 oder 4, vorzugsweise 1 oder 2 Stickstoffatomen im fünfgliedrigen aromatischen Ring. Die fünfgliedrigen aromatischen N-Heterocylen können an den Ringkohlenstoffatomen durch unter den Reaktionsbedingungen inerte Gruppen wie Alkylgruppen, vorzugsweise Methyl und/oder Ethyl, substituiert sein oder zwei benachbarte Kohlenstoffatome des fünfgliedrigen aromatischen N-Heterocyclus können zusammen einem ankondensierten aromatischen carbocyclischen System angehören, wobei das aromatische carbocyclische System seinerseits inerte Gruppen tragen kann. Beispiele für derartige N-Heterocyclen sind die Grundkörper und die substituierten Vertreter der Pyrrole, Pyrazole, Imidazole, Triazole und Tetrazole wie Pyrrol, 2,5-Dimethylpyrrol, Indol, Carbazol, Pyrazol, Indazol, Imidazol, Benzimidazol. Vorzugsweise setzt man Pyrrole und insbesondere alkylsubstituierte Pyrrole, vor allem 2,5-Dimethylpyrrol, ein.

Als Aluminiumalkyl, dessen Alkylgruppen teilweise durch Halogen und/oder Alkoxy ersetzt sein können, kommen Aluminiumalkyle der Formeln AlR₃, AlR₂Hal, AlRHal₂, AlR₂OR', AlRHalOR' und Al₂R₃Hal₃ und deren Gemische in Betracht, in denen R und R' unabhängig voneinander für Methyl, Ethyl oder eine geradekettige oder verzweigte C₃- bis C₈-Alkylgruppe stehen und in denen Hal für Fluor, Brom Iod und vor allem Chlor steht, beispielsweise Trimethylaluminium, Triethylaluminium, Tri-n-propylaluminium, Tri-iso-propylaluminium, Tributylaluminium, Diethylaluminiumchlorid, Diethylaluminiumbromid, Diethylaluminiumethoxid, Diethylaluminiumphenoxid, Ethylaluminiumethoxichlorid. Vorzugsweise werden Aluminiumalkyle vom Typ AlR₃ und AlRHal₂ eingesetzt, wobei Triethylaluminium oder ein Gemisch von Triethylaluminium und Ethylaluminiumdichlorid besonders bevorzugt sind.

Alternativ zu Aluminiumalkylen, deren Alkylgruppen teilweise durch Halogen und/oder Alkoxy ersetzt sind, können auch Gemische aus den entsprechenden Aluminiumalkylen und geeigneten Cokatalysatoren eingesetzt werden, aus denen sich im Reaktor in situ die gewünschten gemischten Aluminiumverbindungen bilden.

Als Cokatalysatoren eignen sich Alkylhalogenide, Alkylsiliciumhalogenide und lewissaure Metallhalogenide, vorzugsweise n-Butylchlorid, n-Butyliodid, Trimethylsilylchlorid, Trimethysilylbromid, Zinntetrachlorid, Germaniumchlorid und vor allem n-Butylbromid.

In dem System aus Aluminiumalkyl, dessen Alkylgruppen teilweise durch Halogen und/oder Alkoxy ersetzt sein können, und Cokatalysator liegen die beiden Komponenten im molaren Verhältnis 1 : 3 bis 30 : 1, vorzugsweise 1 : 1 bis 15 : 1 vor.

Im erfindungsgemäßen Verfahren liegt die Menge der Chromverbindung CrX₃ bzw. des Chromkomplexes CrX₃L normalerweise im Bereich von 1 x 10⁻⁷ bis 1, vorzugsweise von 1 x 10⁻⁶ bis 0,1 und insbesondere von 1 x 10⁻⁵ bis 0,01 mol pro kg der Reaktionsmischung.

Die Menge des fünfgliedrigen aromatischen N-Heterocyclus liegt normalerweise im Bereich von 1 x 10⁻⁸ bis 100, vorzugsweise von 1 x 10⁻⁷ bis 1 und insbesondere von 1 x 10⁻⁵ bis 0,05 mol pro kg der Reaktionsmischung.

Die Menge des Aluminiumalkyls, dessen Alkylgruppen teilweise durch Halogen und/oder Alkoxy ersetzt sein können, liegt normalerweise im Bereich von 1 x 10⁻⁸ bis 500, vorzugsweise von 1 x 10⁻⁷ bis 10 und insbesondere von 5 x 10⁻⁵ bis 0,5 mol pro kg der Reaktionsmischung.

Im erfindungsgemäßen Verfahren ist das Molverhältnis der Komponenten (a), (b) und (c) 1 : 0,1-100 : 0,1-500, vorzugsweise 1 : 0,1-10 : 1-100 und insbesondere 1 : 1-5 : 5-50.

Ganz besonders bevorzugt ist ein Katalysator, der aus (a) [(1,3,5-Tri-n-octyl-1,3,5-triazacyclohexan)CrCl₃] oder [(1,3,5-Tribenzyl-1,3,5-triazacyclohexan)CrCl₃], (b) 2,5-Dimethylpyrrol und (c) Triethylaluminium und Ethylaluminiumdichlorid besteht, wobei diese Komponenten im molaren Verhältnis 1 : 0,1-10 : 0,1-100 und vorzugsweise 1 : 1-5 : 5-50 stehen. Das molare Verhältnis von Triethylaluminium und Ethylaluminiumdichlorid in Komponente (c) beträgt hierbei 1-50:1, vorzugsweise 3-20:1.

Die Oligomerisierung wird vorzugsweise in einem Lösungsmittel durchgeführt. Als Lösungsmittel für die Oligomerisierung können geradkettige, verzweigte oder alicyclische gesättigte Kohlenwasserstoffe mit 1 bis 20 Kohlenstoffatomen wie Butan, Pentan, 3-Methylpentan, Hexan, Heptan, 2-Methylhexan, Octan, Cyclohexan, Methylcyclohexan, 2,2,4-Trimethylpentan, Decalin, geradkettige oder verzweigte halogenierte Kohlenwasserstoffe wie Dichlorethan, aromatische Kohlenwasserstoffe wie Benzol, Toluol, Xylol, Ethylbenzol, Mesitylen, Tetralin und die unter den Reaktionsbedingungen flüssigen oligomeren Reaktionsprodukte wie 1-Hexen selbst eingesetzt werden. Diese Lösungsmittel können entweder einzeln oder als Gemisch verwendet werden.

Im erfindungsgemäßen Verfahren sind bevorzugte Oligomerisierungskatalysatoren weiterhin solche, die als aktivierenden Zusatzstoff ein Alkylalumoxan enthalten.

Dabei sind solche Liganden L ganz besonders bevorzugt, in denen die Gruppen R¹, R² und R³ teilweise oder ganz und unabhängig voneinander für eine Gruppe stehen, welche in der β―Position oder in einer vom N-Atom des 1,3,5-Triazacyclohexanrings noch weiter entfernteren Position einen über ein Kohlenstoffatom gebundenen Substituenten, insbesondere eine Alkyl-, Aryl- oder Silylgruppe trägt. Besonders bevorzugt steht dieser Substituent in der β―Position. Weiterhin besonders bevorzugt handelt es sich bei diesem Substituenten um ein β―alkylsubstituiertes Alkyl, vor allem 2-Ethylhexyl oder 2-n-Propylheptyl. Unter diesen Liganden L sind noch wiederum ganz besonders jene bevorzugt, in denen die Gruppen R⁴, R⁵, R⁶, R⁷, R⁸ und R⁹ für Wasserstoff stehen.

Geeignete Alkylalumoxane sind beispielsweise bekannt aus der DE-A 30 07 725, wobei ihre Strukturen weitgehend unaufgeklärt sind. Es handelt sich um Produkte der vorsichtigen partiellen Hydrolyse von Aluminiumalkylen (vgl. DE-A 30 07 725). Diese Produkte liegen offenbar nicht rein vor, sondern als Gemische von offenkettigen und cyclischen Strukturen des Typs II a und IIb, die miteinander vermutlich im dynamischen Gleichgewicht stehen.

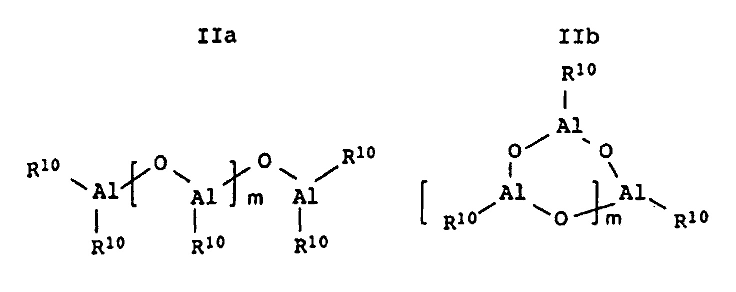

In den Formeln IIa und IIb sind die Gruppen R¹⁰ gleich oder verschieden und stehen unabhängig voneinander für C₁-C₁₂-Alkyl wie Methyl, Ethyl, n-Propyl, i-Propyl, n-Butyl, i-Butyl, sec.-Butyl, tert.-Butyl, n-Pentyl, i-Pentyl, sec.-Pentyl, neo-Pentyl, 1,2-Dimethylpropyl, i-Amyl, n-Hexyl, i-Hexyl, sec.-Hexyl, n-Heptyl, i-Heptyl, n-Octyl, n-Nonyl, n-Decyl, und n-Dodecyl; bevorzugt C₁-C₆-Alkyl wie Methyl, Ethyl, n-Propyl, i-Propyl, n-Butyl, i-Butyl, sec.-Butyl, tert.-Butyl, n-Pentyl, i-Pentyl, sec.-Pentyl, neo-Pentyl, 1,2-Dimethylpropyl, i-Amyl, n-Hexyl, i-Hexyl, sec.-Hexyl, besonders bevorzugt ist Methyl. m ist eine ganze Zahl von 0 bis 40, bevorzugt von 0 bis 25 und besonders bevorzugt von 0 bis 22.

In der Literatur werden auch käfigartige Strukturen für Alumoxane diskutiert (vgl. Organometallics 1996, 15, Seiten 2213-26; Macromol. Symp. 1995, 97, Seiten 15-25).

Ihre Wirksamkeit als aktivierender Zusatzstoff im Sinne der vorliegenden Erfindung erfüllen die Alkylalumoxane unabhängig von ihrer strukturellen Beschaffenheit.

Die verwendete Menge des aktivierenden Alkylalumoxans ist abhängig von dessen Natur. Das Verhältnis von Chromverbindung CrX₃ bzw. Chromkomplex CrX₃L zum aktivierenden Alkylalumoxan beträgt in der Regel 1 : 0,1 bis 1 : 10000, bevorzugt 1 : 1 bis 1 : 1000.

Geeignete Lösungsmittel bei der Verwendung eines Oligomerisierungskatalysators aus CrX₃, einem Liganden L und dem Alkylalumoxan bzw. aus CrX₃L und dem Alkylalumoxan sind aprotische Lösungsmittel, z.B. die weiter oben als Lösungsmittel angeführten aliphatischen oder aromatischen Kohlenwasserstoffe, und vor allem Toluol.

Bevorzugte Olefine für die Oligomerisierung nach dem erfindungsgemäßen Verfahren sind geradkettige und verzweigte α-Olefine mit 2 bis 4 Kohlenstoffatomen sowie deren Gemische und ganz besonders bevorzugt jeweils für sich allein: 1-Propen, 1-Buten und vor allem Ethen, 1-Buten, insbesondere 1-Buten im Gemisch mit seinen Isomeren, wie es etwa im Raffinat II vorliegt.

Insbesondere wegen der Hydrolyseneigung vor allem der Aluminiumverbindungen und gegebenenfalls der Cokatalysatoren ist die Oligomerisierung in der Regel unter weitestgehendem Feuchtigkeitsausschluss durchzuführen. Dabei kommen an sich bekannte Arbeitstechniken zur Anwendung. Vorzugsweise arbeitet man mit ausgeheizten Apparaturen und unter Schutzgas. Als Schutzgase können alle unter den Reaktionsbedingungen chemisch inerten Gase, zweckmäßigerweise Stickstoff oder Argon verwendet werden. Daneben kann das umzusetzende α-Olefin selbst die Funktion des Schutzgases übernehmen, sofern es unter den Reaktionsbedingungen einen hinreichend hohen Dampfdruck hat.

Die Oligomerisierung wird vorzugsweise bei einer Temperatur im Bereich von 1 bis 120 und insbesondere bei 70 bis 110°C und vorzugsweise bei einem Druck im Bereich von 3 bis 120 bar durchgeführt. Der Druck wird dabei zweckmäßigerweise so gewählt, dass bei der eingestellten Temperatur das Einsatzgemisch flüssig vorliegt.

Das erfindungsgemäße Verfahren kann diskontinuierlich oder kontinuierlich durchgeführt werden, wobei im technischen Maßstab der kontinuierlichen Fahrweise der Vorzug zu geben ist.

Für das erfindungsgemäße Verfahren geeignete Reaktoren für die kontinuierliche Reaktionsführung sind dem Fachmann geläufig, etwa aus Ullmann's Enzyklopädie der technischen Chemie, Band 1, 3. Auflage, 1951, Seite 743 ff.; druckfeste Reaktoren sind dort auf Seite 769 ff. beschrieben.

Die übrigen Randbedingungen derartiger Oligomerisierungsreaktionen stellt der Fachmann anhand seines allgemeinen Fachwissens ein, das er etwa der DE-A 196 07 888 entnehmen kann.

Für die Katalysatordesaktivierung bei Umsetzungsende können prinzipiell eine Reihe von Substanzen eingesetzt werden, deren Gemeinsamkeit darin besteht, dass sie in der Lage sind, Aluminiumalkyl-Verbindungen zu hydrolysieren, beispielsweise Wasser und Monoalkohole mit 1 bis 10 Kohlenstoffatomen, wobei diesen Substanzen Mineralsäuren zugesetzt werden können.

Die Produkte der erfindungsgemäßen Oligomerisierung werden zweckmäßigerweise destillativ gereinigt. Auf diese Weise lassen sich etwa aus dem Austrag der Ethen-Oligomerisierung neben der überwiegend aus 1-Hexen bestehenden Haupt(-Hexen-)Fraktion noch eine Decen- und eine Tetradecenfraktion isolieren. Letztere beiden Fraktionen bestehen vorrangig aus verzweigten, internen Olefinen.

Um einen hohen Gesamtumsatz im erfindungsgemäßen verfahren zu erreichen, kann unumgesetztes Ausgangsmaterial zurückgewonnen und in die Umsetzung zurückgeführt werden.

Bevorzugte Produkte des erfindungsgemäßen Verfahrens sind Trimere von α-Olefinen mit 2 bis 4 Kohlenstoffatomen und insbesondere aus Ethen erhältliches 1-Hexen.

Die mit dem erfindungsgemäßen Verfahren erhältlichen Oligomeren eignen sich in besonderem Maße zur Herstellung von Monoalkoholen für Weichmacher und Tenside. Dazu unterwirft man die Oligomeren zweckmäßigerweise der Hydroformylierung, bei der Gemische der um ein Kohlenstoffatom kettenverlängerten Aldehyde und Alkohole entstehen, welche anschließend zu den gewünschten Alkoholen hydriert werden. Die Durchführung der Hydroformylierung und Hydrierung sind dem Fachmann an sich bekannt und bedürfen daher keiner weiteren Erläuterungen (vgl. z.B. Beller et al., Journal of Molecular Catalysis A 104 (1995) Seiten 17-85).

Die folgenden Beispiele erläutern die Erfindung.

### Beispiele

### A) Katalysatoren

2,5-Dimethylpyrrol, CrCl₃(THF)₃, 1,3,5-Tribenzyl-1,3,5-triazacyclohexan und Triethylaluminium wurden von Aldrich Chemical Company Ltd. und Kieselgur von Riedel de Haen AG bezogen.

### Herstellung der 1,3,5-Triazacyclohexane

### Herstellung von 1,3,5-Tri-n-octyl-1,3,5-triazacyclohexan

100 g (0,774 mol) n-Octylamin wurden in kleinen Portionen zu einer auf 0°C gekühlten Suspension von 20,2 g (0,673 mol) Paraformaldehyd in 500 ml Toluol gegeben. Beim anschließenden Erhitzen der Mischung bis zum Siedepunkt ging der Paraformaldehyd in Lösung. Danach wurden Wasser und Toluol abdestilliert. Der Rückstand wurde noch bei 1 mbar von flüchtigen Bestandteilen befreit, mit 100 ml Methanol aufgenommen und über eine 1 cm dicke Schicht von Kieselgel filtriert. Die flüchtigen Anteile des Filtrats wurden anschließend bei 1 mbar entfernt, und es wurden 82,3 g (83 % Ausbeute) der Titelverbindung, als viskose, klare Flüssigkeit erhalten.

Entsprechend wurden folgende 1,3,5-Triazacyclohexane hergestellt:
- 1,3,5-Tri-n-dodecyl-1,3,5-triazacyclohexan
- 1,3,5-Tri-tert.-butyl-1,3,5-triazacyclohexan
- 1,3,5-Triethyl-1,3,5-triazacyclohexan
- 1,3,5-Tris-[(1-phenyl)ethyl]-1,3,5-triazacyclohexan
- 1,3,5-Tris-[(1,1-dimethyl)dodecyl)-2,3,5-triazacyclohexan
- 1,3-Di-n-dodecyl-5-[2-(N,N-dimethylamino)ethyl]-1,3,5-triazacyclohexan
- 1,3,5-Tris-(2-ethylhexyl)-1,3,5-triazacyclohexan
- 1,3,5-Tris-(2-n-propylheptyl)-1,3,5-triazacyclohexan

### Herstellung der Katalysatoren

### Herstellung von "Chrom-Komplex 1": [(1,3,5-Tri-n-octyl-1,3,5-triazacyclohexan)CrCl₃]

662 mg (1,768 mmol) des Tetrahydrofurankomplexes CrCl₃(THF)₃ und 728 mg (1,855 mmol) 1,3,5-Tri-n-octyl-1,3,5-triazacyclohexan (s.o.) wurden bei 25°C in einem Reaktionskolben vorgelegt. Bei -78°C wurden zu dieser Mischung 100 ml über Natrium getrockneter Diethylether einkondensiert. Die so erhaltene Suspension wurde ca. 30 Minuten bei 25°C gerührt. Danach wurde über eine Fritte filtriert und der Filterrückstand solange mit Diethylether gewaschen, bis die Waschlösung nicht mehr grün gefärbt war. Der Rückstand wurde danach bei 25°C und 1 mbar getrocknet, und es wurden so 885 mg der Titelverbindung (98 % Ausbeute) erhalten.

### Herstellung von "Chrom-Komplex 2": [(1,3,5-Tribenzyl-1,3,5-triazacyclohexan)CrCl₃]

In einer ausgeheizten Glasapparatur wurden unter Argon bei 25°C 10 ml trockener Diethylether vorgelegt. 749 mg (20 mmol) des Tetrahydrofurankomplexes CrCl₃(THF)₃ wurden darin suspendiert, und zu der Mischung wurde unter Rühren eine Lösung von 715 mg (20 mmol) 1,3,5-Tribenzyl-1,3,5-triazacyclohexan in 2 ml Diethylether zugetropft. Anschließend wurde die Mischung noch 30 Minuten bei 20°C gerührt und danach unter Argon über eine Fritte filtriert. Der Filterrückstand wurde noch dreimal mit jeweils 10 ml Diethylether gewaschen und dann bei 25°C und 1 mbar getrocknet. Auf diese Weise wurden 0,64 g der Titelverbindung als violetter Feststoff erhalten.

### Herstellung der "Chrom-Komplexe 3 - 10"

Analog zur Herstellung der Komplexe 1 und 2 wurden die folgenden Komplexe von Chrom-(III)-chlorid hergestellt:
"Chrom-Komplex 3":
   [(1,3,5-Tri-n-dodecyl-1,3,5-triazacyclohexan)CrCl₃]
"Chrom-Komplex 4":
   [(1,3,5-Tri-tert.-butyl-1,3,5-triazacyclohexan)CrCl₃]
"Chrom-Komplex 5":
   [(1,3,5-Triethyl-1,3,5-triazacyclohexan)CrCl₃]
"Chrom-Komplex 6":
   [(1,3,5-Tris-[(1-phenyl)ethyl]-1,3,5-triazacyclohexan)CrCl₃]
"Chrom-Komplex 7":
   [(1,3,5-Tris-[(1,1-dimethyl)dodecyl]-1,3,5-triazacyclohexan)CrCl₃]
"Chrom-Komplex 8":
   [(1,3-Di-n-dodecyl-5-[2-(N,N-dimethyl-amino)ethyl]-1,3,5-triazacyclohexan)CrCl₃]
"Chrom-Komplex 9":
   [(1,3,5-Tris-(2-ethylhexyl)-1,3,5-triazacyclohexan)CrCl₃]
"Chrom-Komplex 10":
   [(1,3,5-Tris-(2-n-propylheptyl)-1,3,5-triazacyclohexan)CrCl₃]

### B) Oligomerisierungen

### Beispiel 1: Oligomerisierung von Ethen in Gegenwart von "Komplex 1" und 2,5-Dimethylpyrrol

Ein Stahlautoklav von 100 ml Volumen wurde im Argonstrom bei 105°C 60 Minuten ausgeheizt. Bei 25°C wurde anschließend 14,5 mg "Chrom-Komplex 1" eingefüllt und danach 25 ml über Natrium getrocknetes n-Heptan und 0,5 ml einer Lösung von 143 mg 2,5-Dimethylpyrrol in 10 ml n-Heptan, entsprechend 0,075 mmol 2,5-Dimethylpyrrol. Der Autoklav wurde anschließend dreimal mit Ethen bei Normaldruck durchgespült. Dann erfolgte die Zugabe von 0,75 ml einer 1-molaren Lösung von Triethylaluminium in n-Heptan, woran sich das Aufpressen eines Ethen-Druckes von 25 bar anschloß. Hierauf wurde die Temperatur auf 80°C erhöht und der Ethen-Druck auf 40 bar. Der Autoklaveninhalt wurde 2 Stunden bei diesen Bedingungen gerührt; dann wurde der Autoklav abgekühlt und entspannt. Durch Zugabe von 1 ml Wasser zur Reaktionsmischung wurde der Katalysator desaktiviert. Die im Reaktionsgemisch unlöslichen Bestandteile wurden abgetrennt, getrocknet und gewogen. Berechnet auf 1 g Chrom im Katalysator betrug die Produktivität 18,6 kg. Die relativen Mengen der erhaltenen Produkte wurde gaschromatografisch mit n-Heptan als internem Standard ermittelt:

| | |
|---|---|
| Hexene | 44,4 Gew.-% |
| Decene | 33,1 Gew.-% |
| Tetradecene | 10,9 Gew.-% |
| "Polymere" | 1,5 Gew.-% |

### Beispiele 2 bis 11

Die Beispiele 2 bis 11 wurden analog zu Beispiel 1 durchgeführt. n-Butylbromid (n-BuBr) und Ethylaluminiumdichlorid (EADC) wurden als 0,1 M-Lösungen in n-Heptan eingesetzt. Die Ausgangsstoffe, die zugehörigen Mengenangaben und die Ergebnisse der Versuche sind in der folgenden Tabelle 1 zusammengestellt.

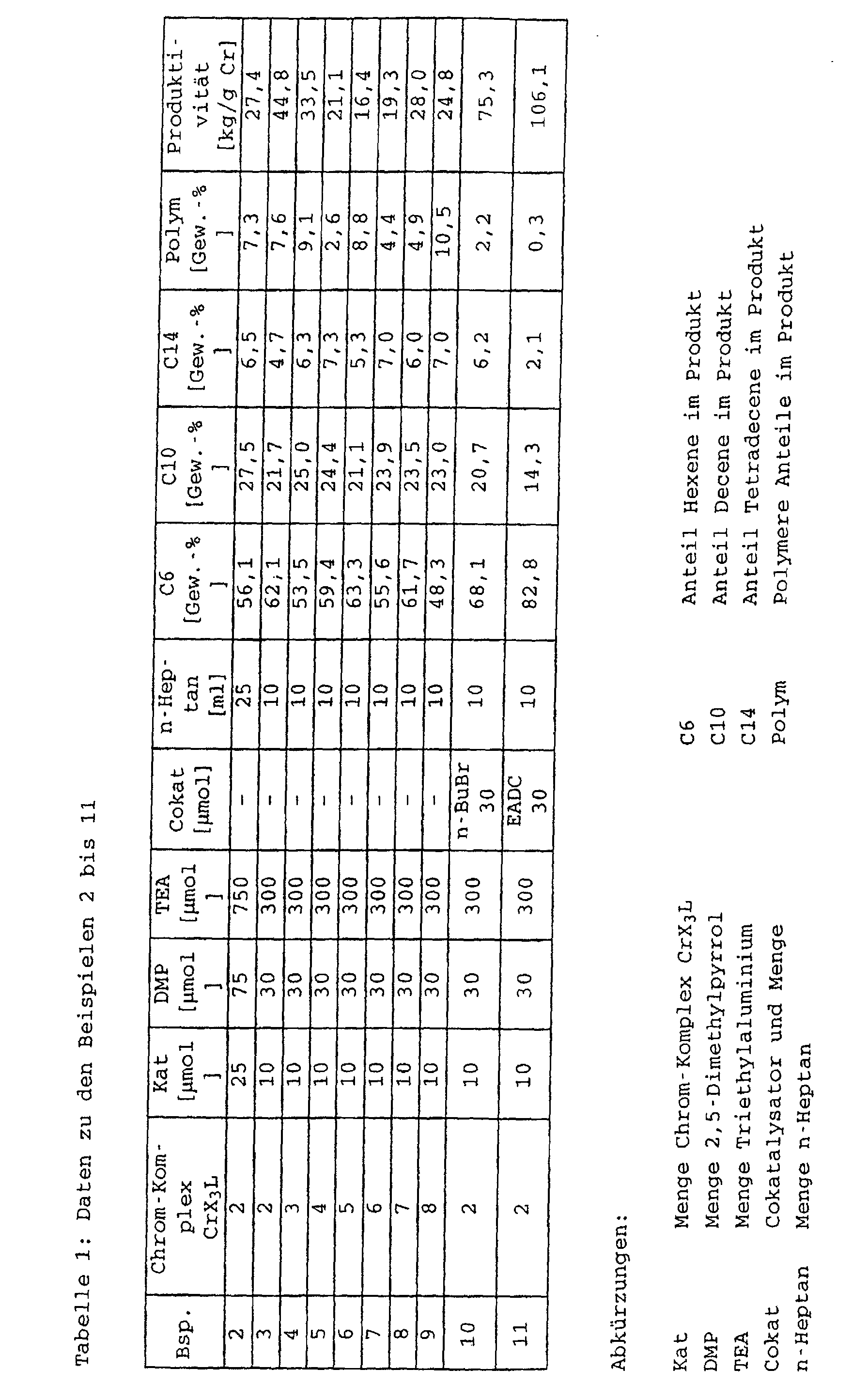

### Beispiel 12: Trimerisierung von 1-Buten in Gegenwart von "Chrom-Komplex 9" und Methylaluminoxan und Hydroformylierung des so erhaltenen Trimeren zum entsprechenden Oxoalkohol

### a) Trimerisierung

Ein Stahlautoklav von 2500 ml Volumen wurde im Argonstrom bei 120°C ausgeheizt. Bei 25°C wurden 750 mg "Chrom-Komplex 9" und 500 g über Natrium getrocknetes Toluol in den Autoklaven gefüllt, und danach wurde der Autoklav dreimal mit 1-Buten gespült. Anschließend wurden noch 50 g einer 1 M Lösung von Methylalumoxan in Toluol und mittels einer Schleuße 500 g 1-Buten in den Autoklaven gegeben. Dann wurde die Temperatur im Autoklaven auf 40°C erhöht und der Druck mit Stickstoff-Gas auf 15 bar eingestellt. Nach zweistündiger Umsetzung unter den so eingestellten Bedingungen wurde der Autoklav abgekühlt und entspannt. Der Katalysator wurde durch Zusatz von 2-Propanol desaktiviert. Im Reaktoraustrag (800 g) wurden bei der anschließenden gaschromatografischen Analyse als Oligomere ausschließlich isomere Dodecene gefunden. Das durch Hydrierung erhaltene Dodecan-Gemisch wies einen ISO-Index von 2,3 auf.

### b1) Hydroformylierung in Gegenwart von Cobaltcarbonyl (Anwendungsbeispiel)

1,06 kg des gemäß Abschnitt a) hergestellten Dodecengemisches wurden mit 4,0 g CO₂(CO)₈ bei 185°C und 280 bar CO/H₂ (1:1) unter Zusatz von 100 g Wasser in einem 2500 ml-Drehrührautoklaven in 5 Stunden hydroformyliert. Der Reaktionsaustrag wurde anschließend mit 10%-iger Essigsäure unter Lufteinleitung bei 90°C oxidativ entkobaltet. Das so erhaltene Oxoprodukt wird in einem 2500 ml-Rohrreaktor in Rieselfahrweise an einem Co/Mo-Festbettkatalysator bei 175oC und 280 bar H₂ unter Zusatz von 10 Gew.-% Wasser, bezogen auf auf die organische Phase, hydriert. Das dabei angefallene Alkoholgemisch wurde destillativ aufgearbeitet und das so isolierte Tridecanol-Gemisch hatte eine OH-Zahl von 279 mg KOH/g. ¹H-NMR-spektroskopisch wurde ein mittlerer Verzweigungsgrad von 2,7 gemessen.

### b2) Hydroformylierung mit Rhodiumbiscarbonylacetylacetonat/Polyethylenimin (Anwendungsbeispiel)

50 mg Rhodiumbiscarbonylacetylacetonat, 4,5 g eines Polyethylenimins der Molmasse M_{w} = 460000, bei dem 60% aller Stickstoffatome mit Laurinsäure amidiert waren, 800 g des gemäß Abschnitt a) hergestellten Dodecengemisches und 196 g Toluol wurden in einem 2500 ml Hubrührautoklaven unter CO/H₂ (1:1) auf 150°C erhitzt. Mittels C0/H₂ wurde ein Druck von 280 bar eingestellt. Nach 7 Stunden wurde der Autoklav abgekühlt, entspannt und entleert. Gaschromatographisch wurde ein Umsatz des Olefins von 93% festgestellt. Das so erhaltene Oxoprodukt wurde in einem 2500 ml-Rohrreaktor in Rieselfahrweise an einem Co/Mo-Festbettkatalysator bei 175°C und 280 bar H₂ unter Zusatz von 10 Gew.-% Wasser, bezogen auf die organische Phase, hydriert. Das dabei angefallene Alkoholgemisch wurde destillativ aufgearbeitet, das erhaltene Tridecanol-Gemisch hatte eine OH- Zahl von 279 mg KOH/g. ¹H-NMR-spektroskopisch wurde ein mittlerer Verzweigungsgrad von 2,9 gemessen.

### Beispiele 13-22: Trimerisierung in Gegenwart von "Chrom-Komplex 9" und Methylaluminoxan

### a) Alternative Herstellung von "Chrom-Komplex 9"

15 ml (+/-)-2-Ethyl-hexylamin (92 mmol) wurden in 200 ml Toluol gelöst, und es wurden 2,75 g Paraformaldehyd (92 mmol) zugegeben. Nach einstündigem Rühren der Mischung wurde das Toluol/Wasser-Azeotrop abdestilliert bis ein Siedepunkt von 110°C erreicht war. Dann wurde im Argonstrom bis unter den Siedepunkt abgekühlt, und es wurden 4,82 g wasserfreies CrCl₃ (31 mmol) zugegeben. Nach erneutem Abdestillieren von einigen ml Toluol und Abkühlen im Argonstrom werden 0,5 g Zink-Pulver zugegeben. Beim weiteren Abdestillieren des Toluols wurde aus der Suspension eine tief-violette Lösung. Toluol wurde noch 30 min abdestilliert und der Rückstand auf 25°C abgekühlt. Nachdem sich das Zink-Pulver abgesetzt hatte wurde die tief-violette Lösung an der Luft abdekantiert und das Lösungsmittel am Rotationsverdampfer entfernt. Der violette, etwas ölige feste Rückstand wurde in Aceton gelöst und filtriert. Die so erhaltene tief-violette Lösung wurde mit Wasser versetzt bis die über dem violetten Niederschlag stehende Lösung nur noch schwach violett gefärbt war. Nach Filtration wurde der violette Rückstand bei zuletzt 50°C im Vakuum (ca. 1 Pa) 40 Stunden lang getrocknet. Es wurden 13,12 g (74 %) des violetten "Chrom-Komplexes 9" erhalten.

### b) Durchführung der Trimerisierungen

Die Trimerisierungen wurden in einem mit Kontaktthermometer, Rührer, Heizpilz und Gaseinleitungrohr versehenen 1 1-Vierhalskolben durchgeführt, in den unter Argon zwischen 30 bis 50 µmol "Chrom-Komplex 9" in 250 ml Toluol bei 40°C vorgelegt wurden.

Methylalumoxan ("MAO") wurde in Form einer 1,6 M Lösung in Toluol verwendet.

Als Borverbindung (nicht erfindungsgemäß) diente Dimethylanilinium-tetrakis-(pentaflourophenyl)-borat ("DMAB"). Nach dessen Zugabe wurde die Reaktionsmischung auf 70°C erhitzt, anschießend auf 40°C abgekühlt und dann mit Triisobutylaluminium ("TIBAL") versetzt.

Durch die nach der Zugabe des MAO bzw. DMAB/TIBAL erhaltene hellgrün/gelbe Lösung wurden 20 bis 40 1/h Ethen bzw. 1-Buten geleitet bzw. es wurde die entsprechende Menge 1-Hexen (nicht erfindungsgemäß) zugetropft.

Während des Versuches über die Reaktionsdauer t wurde die Temperatur konstant bei der Temperatur T gehalten. Die Reaktion wurde durch Zugabe von 15 ml konzentrierte Salzsäure in 50 ml Methanol beendet und die Reaktionsmischung noch 15 min nachgerührt. Danach wurden 250 ml Methanol zugeben und weitere 15 min gerührt. Nach Abfiltrieren von eventuell gebildetem unlöslichem Polymer wurde das Produkt dreimal mit Wasser gewaschen und über Natriumsulfat getrocknet. Ausbeute und Produktverteilung wurden gaschromatographisch aus der so erhaltenen Lösung bestimmt. Weitere Einsatzstoffdaten und die Ergebnisse der Versuche sind Tabelle 2 zu entnehmen.

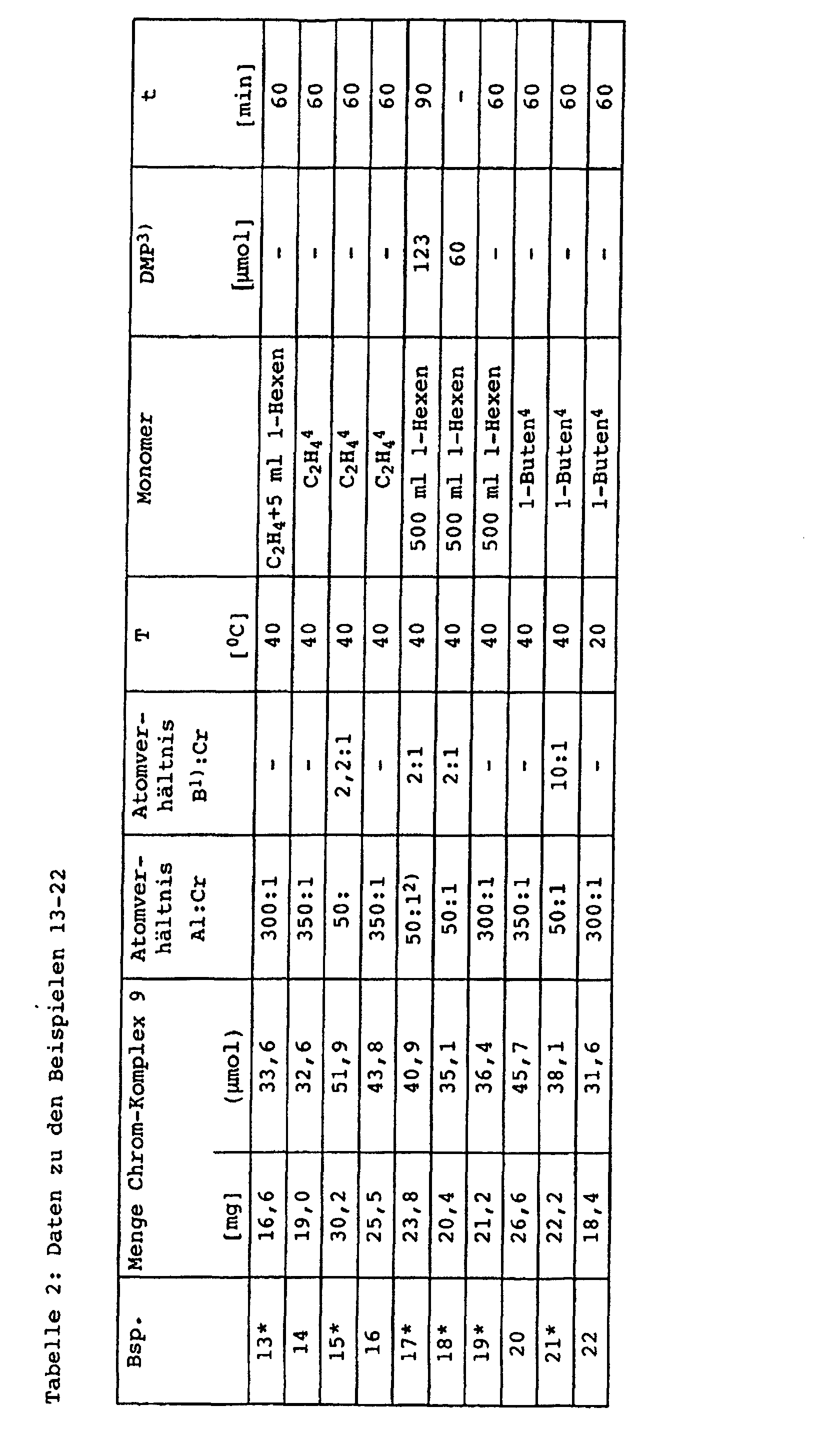

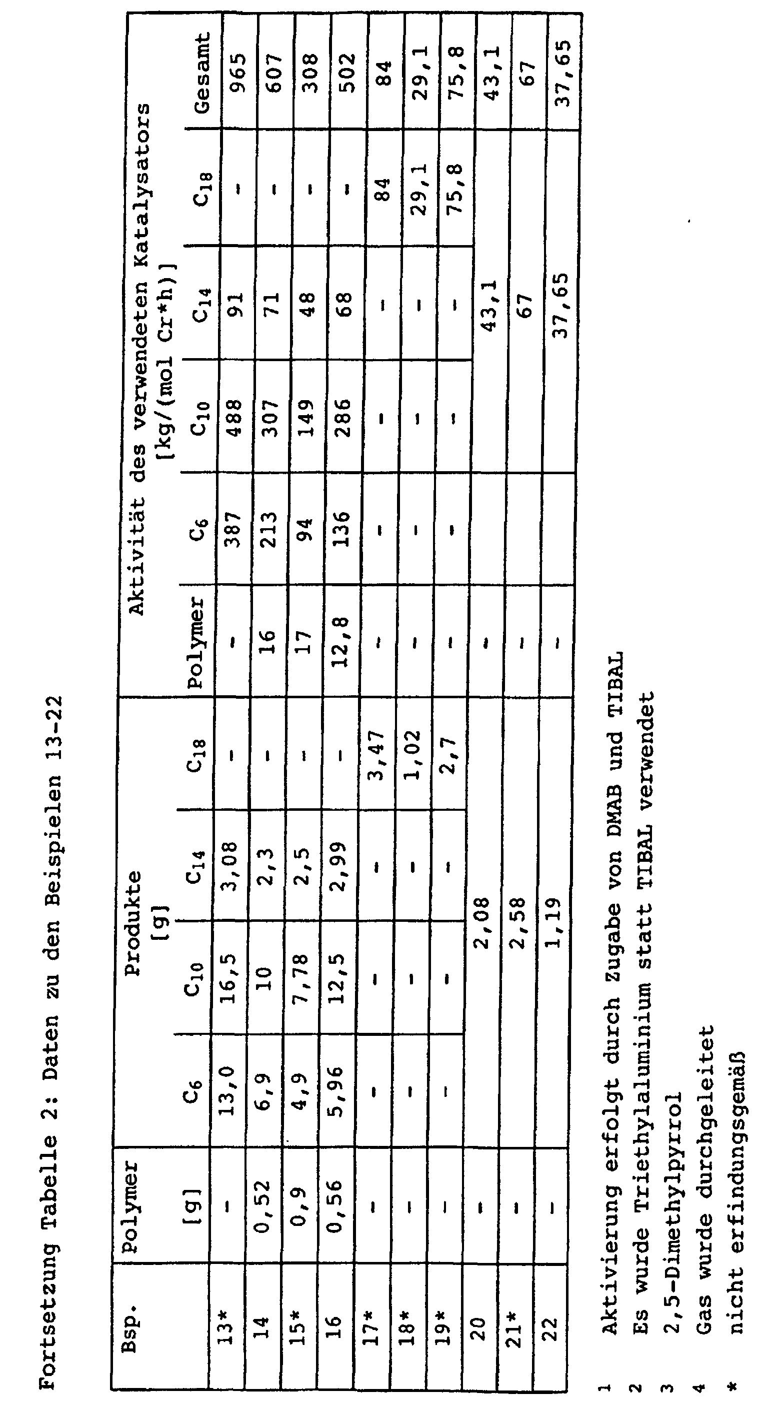

### Beispiele 23 bis 26: Trimerisierung in Gegenwart von "Chrom-Komplex 10" und Methylalumoxan

### a) Alternative Herstellung von "Chrom-Komplex 10"

Die Herstellung erfolgte analog zur alternativen Herstellung von Chrom-Komplex 9 gemäß den Beispielen 13-22.

### b) Durchführung der Trimerisierungen

Die Trimerisierungen der Beispiele 23-25 wurden analog zu den Beispielen 13-22 im Lösungmittel Toluol durchgeführt.

Die Umsetzung gemäß Beispiel 26 erfolgte dagegen in einem 1000 ml-Autoklaven bei einem Druck von 40 bar, ebenfalls in Toluol.

Die weiteren Einsatzstoffdaten und die Ergebnisse der Versuche sind Tabelle 3 zu entnehmen.

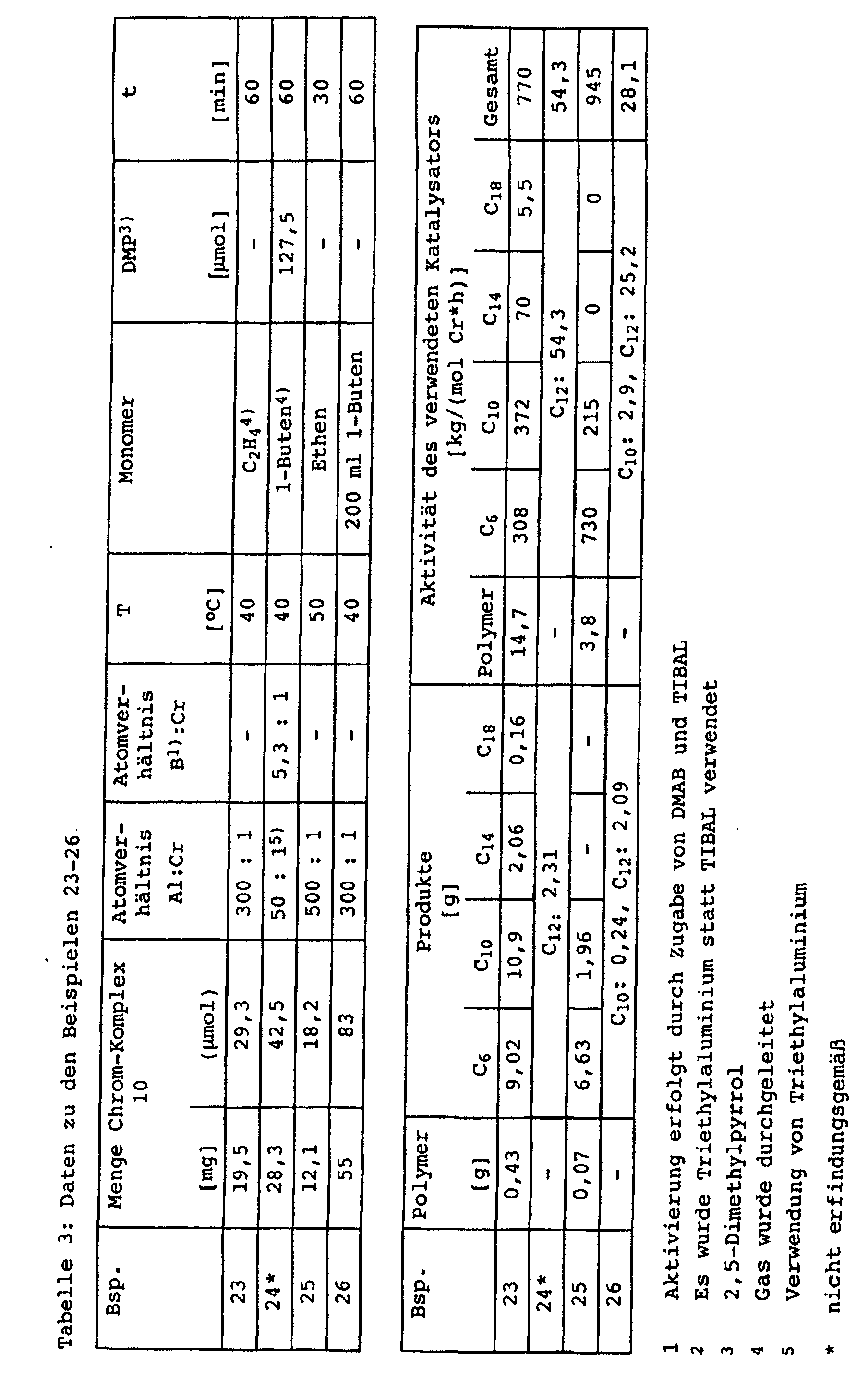

## Patentansprüche

1. Oligomerisierungskatalysator für Olefine, erhältlich aus
a) einer Chromverbindung CrX₃ und der mindestens äquimolaren Menge, bezogen auf die Chromverbindung CrX₃, eines Liganden L oder einem fertigen Chromkomplex CrX₃L, in denen die Gruppen X unabhängig voneinander für abstrahierbare Gegenionen stehen und L für ein 1,3,5-Triazacyclohexan der Formel I steht 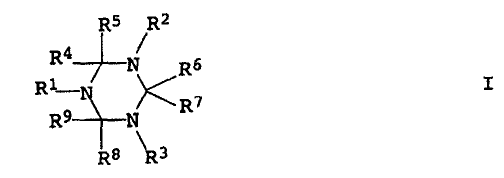 in welcher die Gruppen R¹ bis R⁹ unabhängig voneinander folgende Bedeutungen haben: Wasserstoff oder Si-organische oder gegebenenfalls substituierte C-organische Gruppen mit 1 bis 30 C-Atomen, wobei zwei geminale oder vicinale Reste R¹ bis R⁹ auch zu einem fünf- oder sechsgliedrigen Ring verbunden sein können und
b) mindestens einem aktivierenden Zusatzstoff aus einem gegebenenfalls substituierten fünfgliedrigen aromatischen N-Heterocyclus und mindestens einem Aluminiumalkyl, dessen Alkylgruppen teilweise durch Halogen und/oder Alkoxy ersetzt sein können.

2. Oligomerisierungskatalysator nach Anspruch 1, **dadurch gekennzeichnet, dass** in dem 1,3,5-Triazacyclohexan I die Gruppen R¹, R² und R³ unabhängig voneinander für gegebenenfalls substituiertes C₁- bis C₁₂-Alkyl, C₆- bis C₁₅-Aryl oder C₇- bis C₈-Arylalkyl stehen.

3. Oligomerisierungskatalysator nach Anspruch 1, **dadurch gekennzeichnet, dass** in dem 1,3,5-Triazacyclohexan I die Gruppen R¹, R² und R³ unabhängig voneinander für gegebenenfalls substituiertes C₁- bis C₁₂-Alkyl oder C₇- bis C₈-Arylalkyl stehen.

4. Oligomerisierungskatalysator nach den Ansprüchen 1 bis 3, **dadurch gekennzeichnet, dass** in dem 1,3,5-Triazacyclohexan I die Gruppen R⁴, R⁵, R⁶, R⁷, R⁸ und R⁹ unabhängig voneinander für Wasserstoff oder Methyl stehen.

5. [(1,3,5-Tris-(2-n-propylheptyl)-1,3,5-triazacyclohexan)CrCl₃]

6. [(1,3,5-Tris-(2-ethylhexyl)-1,3,5-triazacyclohexan)CrCl₃]

7. Verfahren zur Herstellung von Oligomeren mit bis zu 30 Kohlenstoffatomen durch Umsetzung eines C₂-C₄-Olefins oder eines Gemischs von C₂-C₄-Olefinen bei Temperaturen von 0 bis 150°C und Drücken von 1 bis 200 bar in Gegenwart eines Oligomerisierungskatalysators, der erhältlich ist aus
a) einer Chromverbindung CrX₃ und der mindestens äquimolaren Menge, bezogen auf die Chromverbindung CrX₃, eines Liganden L oder einem fertigen Chromkomplex CrX₃L, in denen die Gruppen X unabhängig voneinander für abstrahierbare Gegenionen stehen und L für ein 1,3,5-Triazacyclohexan der Formel I steht 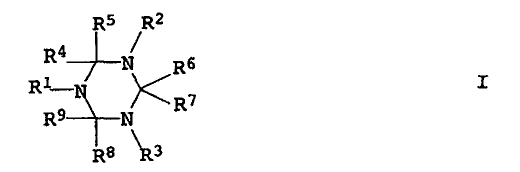 in welcher die Gruppen R¹ bis R⁹ unabhängig voneinander folgende Bedeutungen haben: Wasserstoff oder Si-organische oder gegebenenfalls substituierte C-organische Gruppen mit 1 bis 30 C-Atomen, wobei zwei geminale oder vicinale Reste R¹ bis R⁹ auch zu einem fünf- oder sechsgliedrigen Ring verbunden sein können und
b) mindestens einem aktivierenden Zusatzstoff aus der Gruppe:
i) ein gegebenenfalls substituierter fünfgliedriger aromatischer N-Heterocyclus und mindestens ein Aluminiumalkyl, dessen Alkylgruppen teilweise durch Halogen und/oder Alkoxy ersetzt sein können,
ii) ein Alkylalumoxan.

## Claims

1. An oligomerization catalyst for olefins, obtainable from
a) a chromium compound CrX₃ and the at least equimolar amount, based on the chromium compound CrX₃, of a ligand L or from an existing chromium complex CrX₃L, in which the groups X are, independently of one another, abstractable counterions and L is a 1,3,5-triazacyclohexane of the formula I 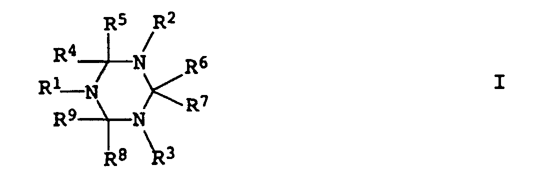 where the groups R¹ to R⁹ are, independently of one another: hydrogen or organosilicon or substituted or unsubstituted carboorganic groups having from 1 to 30 carbon atoms, where two geminal or vicinal radicals R¹ to R⁹ may also be joined to form a five- or six-membered ring, and
b) at least one activating additive comprising a substituted or unsubstituted five-membered aromatic N-heterocycle and at least one aluminum alkyl, some of whose alkyl groups may have been replaced by halogen and/or alkoxy.

2. An oligomerization catalyst as claimed in claim 1, wherein the groups R¹, R² and R³ in the 1,3,5-triazacyclohexane I are, independently of one another, substituted or unsubstituted C₁-C₁₂-alkyl, C₆-C₁₅-aryl or C₇-C₈-arylalkyl.

3. An oligomerization catalyst as claimed in claim 1, wherein the groups R¹, R² and R³ in the 1,3,5-triazacyclohexane I are, independently of one another, substituted or unsubstituted C₁-C₁₂-alkyl or C₇-C₈-arylalkyl.

4. An oligomerization catalyst as claimed in any of claims 1 to 3, wherein the groups R⁴, R⁵, R⁶, R⁷, R⁸ and R⁹ in the 1,3,5-triazacyclohexane I are, independently of one another, hydrogen or methyl.

5. [(1,3,5-Tris(2-n-propylheptyl)-1,3,5-triazacyclohexane)CrCl₃]

6. [(1,3,5-Tris(2-ethylhexyl)-1,3,5-triazacyclohexane)CrCl₃]

7. A process for preparing oligomers having up to 30 carbon atoms by reaction of a C₂-C₄-olefin or a mixture of C₂-C₄-olefins at from 0 to 150°C and pressures of from 1 to 200 bar in the presence of an oligomerization catalyst obtainable from
a) a chromium compound CrX₃ and the at least equimolar amount, based on the chromium compound CrX₃, of a ligand L or from an existing chromium complex CrX₃L, in which the groups X are, independently of one another, abstractable counterions and L is a 1,3,5-triazacyclohexane of the formula I 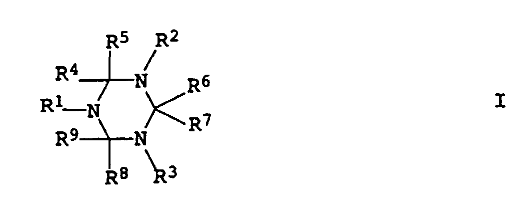 where the groups R¹ to R⁹ are, independently of one another: hydrogen or organosilicon or substituted or unsubstituted carboorganic groups having from 1 to 30 carbon atoms, where two geminal or vicinal radicals R¹ to R⁹ may also be joined to form a five- or six-membered ring, and
b) at least one activating additive from the group
i) an unsubstituted or substituted five-membered aromatic N-heterocycle and at least one aluminium alkyl, some of whose alkyl groups may have been replaced by halogen and/or alkoxy,
ii) an alkylalumoxane.

## Revendications

1. Catalyseur d'oligomérisation pour des oléfines, pouvant être obtenu à partir
a) d'un composé du chrome CrX₃ et de la quantité au moins équimolaire, par rapport au composé du chrome CrX₃, d'un ligand L ou d'un complexe du chrome CrX₃L préparé, dans lesquels les groupes X représentent, indépendamment les uns des autres, des contre-ions déplaçables et L représente un 1,3,5-triazacyclohexane de formule I 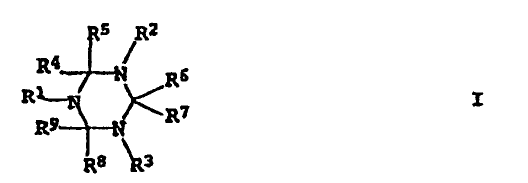 dans laquelle les groupes R¹ à R⁹ ont, indépendamment les uns des autres, la signification suivante : un atome d'hydrogène ou des groupes Si organiques ou C organiques éventuellement substitués possédant 1 à 30 atomes de C, deux groupes R¹ à R⁹ géminés ou vicinaux pouvant être également liés à un noyau à cinq ou six éléments et
b) au moins un additif d'activation composé d'un N-hétérocycle aromatique à cinq éléments éventuellement substitué et d'au moins un alkylure d'aluminium dont les groupes alkyle peuvent être partiellement substitués par un atome d'halogène et/ou un groupe alcoxy.

2. Catalyseur d'oligomérisation selon la revendication 1, **caractérisé en ce que**, dans le 1,3,5-triazacyclohexane I, les groupes R¹, R² et R³ représentent, indépendamment les uns des autres, un groupe alkyle en C₁ à C₁₂ éventuellement substitué, un groupe aryle en C₆ à C₁₅ ou un groupe arylalkyle en C₇ à C₈.

3. Catalyseur d'oligomérisation selon la revendication 1, **caractérisé en ce que**, dans le 1,3,5-triazacyclohexane I, les groupes R¹, R² et R³ représentent, indépendamment les uns des autres, un groupe alkyle en C₁ à C₁₂ éventuellement substitué ou un groupe arylalkyle en C₇ à C₈.

4. Catalyseur d'oligomérisation selon les revendications 1 à 3, **caractérisé en ce que**, dans le 1,3,5-triazacyclohexane I, les groupes R⁴, R⁵, R⁶, R⁷, R⁸ et R⁹ représentent, indépendamment les uns des autres, un atome d'hydrogène ou un groupe méthyle.

5. [(1,3,5-tris-(2-n-propylheptyl)-1,3,5-triazacyclohexane)CrCl₃]

6. [(1,3,5-tris-(2-éthylhexyl)-1,3,5-triazacyclohexane)CrCl₃]

7. Procédé de préparation d'oligomères possédant jusqu'à 30 atomes de carbone au moyen de la réaction d'une oléfine en C₂ à C₄ ou d'un mélange d'oléfines en C₂ à C₄, à une température allant de 0°C à 150°C et sous des pressions allant de 1 bar à 200 bars, en présence d'un catalyseur d'oligomérisation pouvant être obtenu à partir
a) d'un composé du chrome CrX₃ et de la quantité au moins équimolaire, par rapport au composé du chrome CrX₃, d'un ligand L ou d'un complexe du chrome CrX₃L préparé, dans lesquels les groupes X représentent, indépendamment les uns des autres, des contre-ions déplaçables et L représente un 1,3,5-triazacyclohexane de formule I 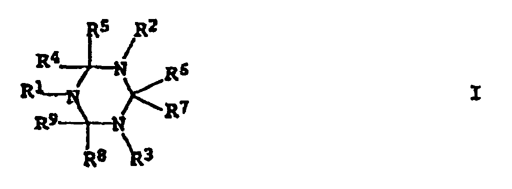 dans laquelle les groupes R¹ à R⁹ ont, indépendamment les uns des autres, la signification suivante : un atome d'hydrogène ou des groupes Si organiques ou C organiques éventuellement substitués possédant 1 à 30 atomes de C, deux groupes R¹ à R⁹ géminés ou vicinaux pouvant être également liés à un noyau à cinq ou six éléments et
b) au moins un additif d' activation du groupe formé par :
i) un N-hétérocycle aromatique à cinq éléments éventuellement substitué et d'au moins un alkylure d'aluminium dont les groupes alkyle peuvent être partiellement substitués par un atome d'halogène et/ou un groupe alcoxy,
ii) un alkylaluminoxane.
